# EUROPEAN PATENT APPLICATION

(11) **EP 4 266 825 A1**
(43) Date of publication of application: **25.10.2023**
(21) Application number: 23165768.5
(22) Date of filing: 31.03.2023
(51) Int. Cl.: H05B 3/04, H05B 3/12, H05B 3/16, H05B 3/26, A24F 40/10, A24F 40/46, A61M 11/04

(54) **ATOMIZATION CORE, ATOMIZER, AND ELECTRONIC ATOMIZATION DEVICE**

(30) Priority: 20.04.2022 CN 202210418914
(71) Applicant: Shenzhen Smoore Technology Limited, Shenzhen, Guangdong 518102 (CN)
(72) Inventor: LV, Hongxia, Shenzhen, Guangdong 518102 (CN); LI, Pei, Shenzhen, Guangdong 518102 (CN); JIANG, Zhenlong, Shenzhen, Guangdong 518102 (CN)
(74) Representative: De Arpe Tejero, Manuel

(57) **Abstract**

The present disclosure discloses an atomization core (10), an atomizer (100), and an electronic atomization device (300). The atomization core (10) includes a liquid conducting substance (11), a heating layer (12), and an oxide layer (13). The liquid conducting substance (11) has an atomization surface (111), the heating layer (12) is disposed on the atomization surface (111) of the liquid conducting substance (11), and the oxide layer (13) is disposed on a surface of the heating layer (12) away from the liquid conducting substance (11). The heating layer (12) is disposed on the atomization surface (111) of the liquid conducting substance (11), and the oxide layer (13) is disposed on the surface of the heating layer (12) away from the liquid conducting substance (11), so as to protect the heating layer (12) of the atomization core (10), which avoid affecting the heating performance of the atomization core (10).

## Description

### TECHNICAL FIELD

The present disclosure relates to the technical field of atomization technology, and in particular to an atomization core, an atomizer, and an electronic atomization device.

### BACKGROUND

An electronic atomization device is generally composed of an atomizer and a power supply assembly. The power supply assembly is configured to supply power to the atomizer, and the atomizer heats and atomizes an aerosol-forming substance after a power being applied to generate an aerosol for a user to suck. The atomization core includes a liquid conducting substance and a heating element. A heating atomization process of the atomizer is realized mainly by heating through the heating element of the atomization core after a power being applied, so as to heat and atomize the aerosol-forming substance.

Generally, the heating element of the atomization core is a metal heating film, but in the process of the atomization, the metal heating film may oxidize and fail when the aerosol-forming substance supply is insufficient, which affects the stability and the service life of the product.

### SUMMARY

The present disclosure mainly provides an atomization core, an atomizer, and an electronic atomization device, to overcome the issue that a metal heating film on an atomization core is easy to fail and has a short life in an atomization process in the prior art.

In order to overcome the foregoing technical problem, a first technical solution provided in the present disclosure is an atomization core. The atomization core includes a liquid conducting substance, a heating layer, and an oxide layer. The liquid conducting substance has an atomization surface, the heating layer is disposed on the atomization surface of the liquid conducting substance, and the oxide layer is disposed on a surface of the heating layer away from the liquid conducting substance.

The oxide layer includes aluminum oxide and/or silicon oxide.

The thickness of the oxide layer ranges from 200 nm to 600 nm.

The oxide layer is formed on the surface of the heating layer away from the liquid conducting substance through physical vapor deposition.

The atomization core further includes two electrodes, and the two electrodes are disposed on the surface of the heating layer away from the liquid conducting substance. The oxide layer and the two electrodes jointly cover the heating layer.

The two electrodes dense substance having holes are disposed at intervals on the surface of the side of the heating layer away from the liquid conducting substance and are respectively located on two sides of the oxide layer, and the oxide layer covers a part of the heating layer that is not covered by the two electrodes.

The two electrodes both contact the oxide layer, the heating layer, and the liquid conducting substance.

The two electrodes both contact the surface of the side of the heating layer away from the liquid conducting substance, the side surfaces of the oxide layer, the side surfaces of the heating layer and the surface of the side of the liquid conducting substance to prevent a gap from existing between the electrode and the oxide layer when the electrode is disposed on two sides of the oxide layer.

The oxide layer completely cover the surface of the heating layer away from the liquid conducting substance and the side surface of the heating layer; two through holes spaced apart from each other are disposed on the oxide layer by forming a hole, and the two electrodes are electrically connected to the heating layer through the two through holes in the oxide layer, respectively.

The thickness of the oxide layer is less than the thickness of the electrode.

The heating layer is a porous heating film.

The oxide layer is a porous structure.

The liquid conducting substance is a porous ceramic substance or a dense substance having holes.

In order to overcome the foregoing technical problem, a second technical solution provided in the present disclosure is an atomizer. The atomizer includes a liquid storage cavity configured to store an aerosol-forming substance and the atomization core of any of the above. The atomization core is configured to absorb, heat, and atomize the aerosol-forming substance in the liquid storage cavity.

In order to overcome the foregoing technical problem, a third technical solution provided in the present disclosure is an electronic atomization device. The electronic atomization device includes a power supply assembly and the atomizer of any of the above. The power supply assembly provides energy for the atomizer.

Beneficial effects of the present disclosure are as follows. Different from the prior art, the present disclosure discloses an atomization core, an atomizer, and an electronic atomization device. The atomization core includes a liquid conducting substance, a heating layer, and an oxide layer. The liquid conducting substance has an atomization surface, the heating layer is disposed on the atomization surface of the liquid conducting substance, and the oxide layer is disposed on a surface of the heating layer away from the liquid conducting substance. The oxide layer is disposed on the surface of the heating layer away from the liquid conducting substance, so that the oxide layer protects the heating layer in the process of heating and atomization, and avoids the failure of the heating layer due to oxidation in the process of the atomization, thereby the stability of the heating layer is improved, and the service life of the heating layer is further increased.

### BRIEF DESCRIPTION OF THE DRAWINGS

In order to describe the technical solutions in embodiments of the present disclosure or the related art more clearly, the following briefly describes the accompanying drawings required for describing the embodiments or the related art. Apparently, the accompanying drawings in the following description show only some embodiments of the present disclosure, and those of ordinary skill in the art may still derive other accompanying drawings from the accompanying drawings without creative efforts.
FIG. 1 is a schematic structural diagram of an electronic atomization device according to the present disclosure.
FIG. 2 is a schematic structural diagram of an atomizer of the electronic atomization device provided in FIG. 1.
FIG. 3 is a schematic structural diagram of a first embodiment of the atomization core in FIG. 2.
FIG. 4 is a schematic structural top view of the atomization core provided in FIG. 3.
FIG. 5 is a schematic structural diagram of a second a second embodiment of the atomization core in FIG. 2.
FIG. 6 is a schematic structural diagram of a third embodiment of the atomization core in FIG. 2.

### DETAILED DESCRIPTION

The technical solutions in embodiments of the present disclosure are clearly and completely described below with reference to the accompanying drawings in the embodiments of the present disclosure. Apparently, the described embodiments are merely some rather than all of the embodiments of the present disclosure. All other embodiments derived by a person of ordinary skill in the art based on the embodiments of the present disclosure without creative efforts shall fall within the protection scope of the present disclosure.

The terms "first", "second", and "third" in the embodiments of the present disclosure are merely intended for a purpose of description, and shall not be understood as an indication or implication of relative importance or implicit indication of the number of indicated technical features. Therefore, features defining "first", "second", and "third" may explicitly or implicitly include at least one of the features. In description of the present disclosure, "a plurality of" means at least two, such as two and three, unless otherwise specifically defined. In addition, the terms "include", "have", and any variant thereof are intended to cover a non-exclusive inclusion. For example, a process, a method, a system, a product, or a device that includes a series of steps or units is not limited to the listed steps or units, and instead, further optionally includes a step or unit that is not listed, or further optionally includes another step or unit that is intrinsic to the process, method, product, or device.

Embodiments mentioned in the specification mean that particular features, structures, or characteristics described with reference to the embodiments may be included in at least one embodiment of the present disclosure. The term appearing at different positions of this specification may not be the same embodiment or an independent or alternative embodiment that is mutually exclusive with other embodiments. A person skilled in the art explicitly or implicitly understands that the embodiments described in the specification may be combined with other embodiments.

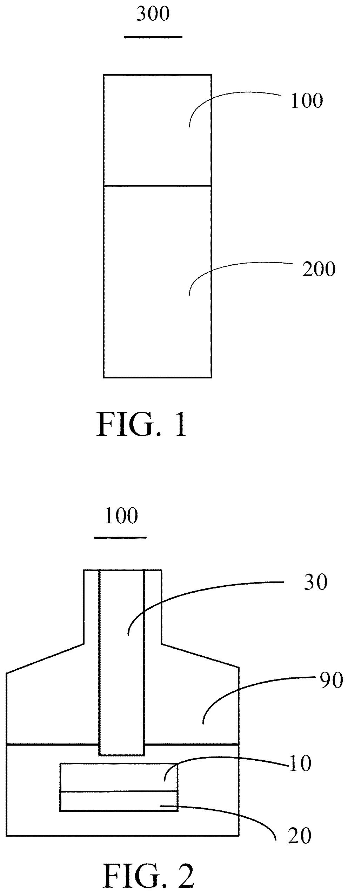

Referring to FIG. 1 and FIG. 2, FIG. 1 is a schematic structural diagram of an electronic atomization device according to the present disclosure, and FIG. 2 is a schematic structural diagram of an atomizer in the electronic atomization device provided in FIG. 1.

Referring to FIG. 1, the present disclosure provides an electronic atomization device 300. The electronic atomization device 300 includes an atomizer 100 and a power supply assembly 200. The power supply assembly 200 is configured to provide energy to the atomizer 100, and the atomizer 100 is configured to heat and atomize an aerosol-forming substance after a power being applied to generate an aerosol for a user to suck.

Optionally, the atomizer 100 and the power supply assembly 200 in the electronic atomization device 300 may be integrally formed or may be detachably connected, which may be designed according to a specific requirement.

Referring to FIG. 2, the atomizer 100 includes a liquid storage cavity 90, an air outlet channel 30, an atomization core 10, and an atomization cavity 20 formed in the atomizer 100. The liquid storage cavity 90 is configured to store an aerosol-forming substance, and the atomization core 10 is configured to adsorb the aerosol-forming substance in the liquid storage cavity 90, and heat and atomize the absorbed aerosol-forming substance to generate an aerosol finally. The aerosol generated by the atomization is in the atomization cavity 20 and flows through the air outlet channel 30 with an external airflow, and finally flows out of the atomizer 100 to be sucked by the user.

A heating element of the atomization core is generally a metal heating film. Nano particles of the metal heating film are prone to oxidation and failure during sintering and atomization, in the case of insufficient aerosol-forming substance supply. For the problem that the metal heating film layer is prone to oxidation and failure, a protective layer formed by precious metals such as gold and platinum is generally disposed on the surface of the metal heating film in the prior art to overcome the foregoing issue. However, the particles made of gold and platinum are prone to overheat when the amount of aerosol-forming substance is small, which causing reunion of the precious metal particles, and the metal heating film is exposed to the air and oxidizes and fails. In view of this, the present disclosure provides an atomization core 10. Details are described as follows.

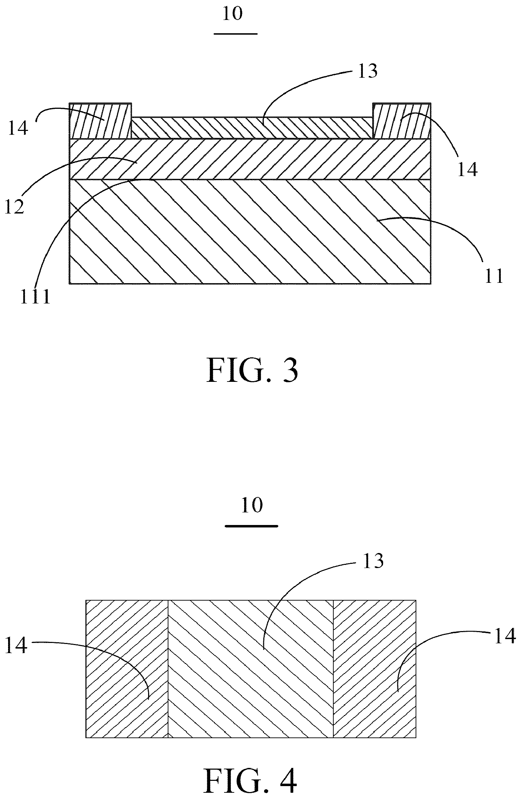

Referring to FIG. 3 and FIG. 4, FIG. 3 is a schematic structural diagram of a first embodiment of an atomization core in FIG. 2, and FIG. 4 is a schematic structural top view of the atomization core provided in FIG. 3.

The atomization core 10 includes a liquid conducting substance 11, a heating layer 12, and an oxide layer 13. The liquid conducting substance 11 has an atomization surface 111, the heating layer 12 is disposed on the atomization surface 111 of the liquid conducting substance, and the oxide layer 13 is disposed on a surface of a side of the heating layer 12 away from the liquid conducting substance 11. The oxide layer 13 is disposed on the surface of the side of the heating layer 12 away from the liquid conducting substance 11 to protect the heating layer 12 from directing contact between the heating layer 12, and the air is isolated to avoid leading to the failure of the heating layer 12 due to the oxidation of the heating layer 12 in a heated environment, which can help improve the stability of the heating layer 12, and prolong the service life of the heating layer 12.

In the embodiment, the oxide layer 13 may be made of aluminum oxide or silicon oxide or a mixture of the aluminum oxide and the silicon oxide. The oxide layer 13 is made of oxides and has strong antioxidant capacity, and both the aluminum oxide and the silicon oxide are high oxides with stable performance. Therefore, the oxide layer 13 is not prone to an oxidation reaction to change the performance when contacting the air during the atomization, thereby ensuring the stability of the atomization core 10. In addition, a melting point and a boiling point of the aluminum oxide and the silicon oxide are relatively high, and have strong resistance to high temperature. During the atomization, even if overheat occurs when the aerosol-forming substance in the atomization core 10 is insufficient, particle aggregation does not occur on the oxide layer 13 due to the overheat, resulting in the failure of the atomization core 10. The problem of the failure of the atomization core 10 as a result of the precious metal particle reunion caused by the overheat of the precious metal materials when the protective layer is made of precious metal materials such as gold and platinum and a small number of aerosol-forming substances exist in the atomization core 10 in the prior art is effectively solved, which improves the stability of the atomization core 10, and prolongs the service life of the atomization core 10. In addition, compared with the protective layer made of the precious metal material, the protective layer of the heating layer 12 made of the oxide has lower costs, which effectively saves the production cost of the atomizer 100.

The oxide layer 13 is prepared by depositing the oxide on the surface of the side of the heating layer 12 away from the liquid conducting substance 11. In the embodiment, the oxide layer 13 is prepared by sputtering the oxide by using a sputtering process. Optionally, the sputtering process may be a DC sputtering process, an AC sputtering process, a magnetron sputtering process, or the like. The aluminum oxide and the silicon oxide of the oxide layer 13 are materials having high density. However, since the oxide layer 13 is prepared on the surface of the heating layer 12 by the sputtering process, the structure of the oxide layer 13 is also a porous structure due to the production process.

The thickness of the oxide layer 13 ranges from 200 nm to 600 nm, so as to ensure that oxide layer 13 can better protect the heating layer 12. It may be understood that if the thickness of the oxide layer 13 is excessively small, the structural strength of the oxide layer 13 is relatively low, the ability to block air is also weakened, and the protective effect on the heating layer 12 is weakened accordingly, which causes the air to still contact the heating layer 12, so that the heating layer 12 is oxidized, leading to the failure of the atomization core 10. In addition, the inventor of the present disclosure found that oxide layer 13 should not be excessively thick. On the one hand, the thermal conductivity of the oxide layer 13 is less than that the thermal conductivity of the metal material. If the thickness of the oxide layer 13 is excessively large, a heating rate of the atomization surface 111 may be affected and an amount of the aerosol generated by the atomization is also affected. On the other hand, since the atomization surface 111 is a porous structure, excessively thick of the oxide layer 13 may block the porous structure and reduce the liquid guiding rate of aerosol-forming substance, and then cause problems such as an abnormal high temperature and dry burning.

In other embodiments, the oxide layer 13 may also be manufactured by using other process technologies, so as to protect the heating layer 12.

A shape and a size of the liquid conducting substance 11 are not limited. The liquid conducting substance 11 is made of a material having a porous structure, for example, the liquid conducting substance 11 may be made of porous ceramic,porous glass, porous plastic, porous metal, and the like. Or, the liquid conducting substance 11 is made of a dense material having holes, for example, glass with holes, porous plastic with holes, metal with holes, and the like. In the embodiment, the material of the liquid conducting substance 11 is a porous ceramic substance. The porous ceramic has pores functioning to guide and store liquid, so that the aerosol-forming substance in the liquid storage cavity 90 is absorbed by the liquid conducting substance 11 and then permeates to the atomization surface 111 for heating and atomization. In addition, the porous ceramic has a stable chemical property, and does not produce chemical reaction with the aerosol-forming substance, and the porous ceramic can bear high temperature, and is not deformed due to the excessively high heating temperature during the atomization. The porous ceramic is an insulator and is not electrically connected to the heating layer 12 on the surface, and may not cause the failure of the atomization core 10 due to a short circuit, and the porous ceramic is easy to manufacture and low in cost. In the embodiment, the liquid conducting substance 11 is a rectangular porous ceramic.

In some embodiments, the porosity of the porous ceramic ranges from 30% to 70%. The porosity is a ratio of a total volume of tiny gaps in a porous medium to a total volume of the porous medium. A value of the porosity may be adjusted according to the composition of the aerosol-forming substance. For example, when the viscosity of the aerosol-forming substance is large, a higher porosity is selected to ensure the liquid guiding effect.

In others embodiments, the porosity of the porous ceramic ranges from 50% to 60%. The porosity of the porous ceramic ranges from 50% to 60%. On the one hand, the porosity may be ensured that the porous ceramic has better liquid guiding efficiency and prevent the phenomenon of dry burning due to unsmooth flow of the aerosol-forming substance, so as to improve the atomization effect of the atomizer 100; and on the other hand, the porosity of the porous ceramic may be prevented from being excessively large, the liquid is guided too fast and is difficult to lock, resulting in an increased probability of the liquid leakage, and affecting the performance of the atomizer 100.

In other embodiments, when the liquid conducting substance 11 is made of other materials with the porous structure, the ratio of the porosity in the liquid conducting substance 11 may be set according to the setting form on the porous ceramic. Details are not described herein again in the present disclosure.

It may be understood that when the liquid conducting substance 11 is glass with holes, plastic with holes, or metal with holes, the liquid conducting substance 11 may be made of the glass, the plastic, or the metal formed by forming a hole on a dense glass substance, plastic substance, or metal substance.

When the liquid conducting substance 11 is the metal with holes, an insulating layer is disposed between the liquid conducting substance 11 and the heating layer 12. The insulating layer is configured to insulate the liquid conducting substance 11 and the heating layer 12 to avoid the short circuit caused by the electrical connection between the liquid conducting substance 11 and the heating layer 12.

The heating layer 12 is disposed on the atomization surface 111 of the liquid conducting substance 11, and generates heat under the power-on state to heat and atomize the aerosol-forming substance. Optionally, the heating layer 12 may be at least one of a heating film, a heating coating, a heating circuit, a heating sheet, or a heating mesh. In the embodiment, the heating layer 12 is a porous heating film structure. It can be understood that the porous structure on the heating layer 12 allows the liquid aerosol-forming substance to permeate into the surface of the heating layer 12 or the atomization surface 111 more efficiently, so as to improve the liquid guiding efficiency and thermal conductivity of the heating layer 12 and improve the atomization effect of the atomization core 10.

The heating layer 12 may be made of a material allowing stable combination with the liquid conducting substance 11. For example, the heating layer 12 may be made of a material such as titanium, zirconium, a titanium-aluminum alloy, a titanium-zirconium alloy, a titanium-molybdenum alloy, a titanium-niobium alloy, an iron-aluminum alloy, a tantalum-aluminum alloy, stainless steel.

Titanium and zirconium have the following characteristics. Titanium and zirconium are both biocompatible metals, and titanium is also a pro-biotic metal element with higher safety. Titanium and zirconium have larger resistivity among metallic materials, and have three times the original resistivity after alloying in certain proportion at room temperature, and therefore titanium-zirconium alloy are more suitable to be the material of the heating layer 12. Titanium and zirconium have small coefficients of thermal expansion and have smaller coefficients of thermal expansion after alloying, and have a better heat match with the porous ceramic. After alloying in certain proportion, a melting point of the titanium-zirconium alloy is lower, and a film-forming property of the magnetron sputtering is better. After the metal coating, it can be seen through electron microscope analysis that microscopic particles are spherical, and the particles gather together to form a microscopic morphology similar to cauliflower. It can be seen through the electron microscopic analysis that microscopic particles of the film formed by the titanium-zirconium alloy are flaky, a part of grain boundaries between the particles disappears, and the continuity is better. Titanium and zirconium both have good plasticity and elongation, and the resistance to a thermal cycle and current impacting of the titanium-zirconium alloy film is better. Titanium is often used as a stress buffer layer for the metal and the ceramic and an activating element for ceramic metallization. Titanium reacts with a ceramic interface to form a relatively strong chemical bond, so as to improve an adhesive force of the titanium-zirconium alloy film. Based on the above characteristics of titanium and zirconium, in the embodiment, the heating layer 12 is made of the titanium-zirconium alloy.

The thickness of the heating layer 12 ranges from 0.1 µm to 10 µm. In the embodiment, the thickness of the heating layer 12 may be any specific value of 0.1 µm, 1 µm, 2 µm, 3 µm, 4 µm, 5 µm, 6 µm, 7 µm, 8 µm, 9 µm, or 10 µm. Preferably, the thickness of the heating layer 12 ranges from 2 µm to 5 µm, the thickness can ensure that the thickness of the heating layer 12 matches a pore size of the liquid conducting substance 11, so as to prevent the heating layer 12 from blocking the micro-pore for guiding and storing liquid in the liquid conducting substance 11, thereby improving the stability of liquid supply in the atomization process of the atomization core 10, and increasing the service life of the atomization core 10.

Optionally, the heating layer 12 may be manufactured on the atomization surface 111 of the liquid conducting substance 11 by using a process such as physical vapor deposition or chemical vapor deposition. For example, the heating layer 12 may be manufactured by using process technologies such as sputtering, evaporation coating, atomic layer deposition, and the like.

In the embodiment, the titanium-zirconium alloy film made of the titanium-zirconium alloy is a locally dense film, but the titanium-zirconium alloy film formed on the surface of the liquid conducting substance 11 also becomes a porous continuous structure since the liquid conducting substance 11 is the porous structure, and a pore size of the titanium-zirconium alloy film is slightly smaller than a pore size of the micro-pore on the surface of the liquid conducting substance 11.

Referring to FIG. 3, in the embodiment, the atomization core 10 further includes two electrodes 14. The two electrodes 14 are respectively electrically connected to the power supply assembly 200 in the electronic atomization device 300, and the two electrodes 14 are configured to supply power to the heating layer 12 of the atomization core 10, so that the heating layer 12 generates heat under the power-on state, and then heats and atomizes the aerosol-forming substance absorbed in the liquid conducting substance 11 to generate the aerosol.

In the embodiment, as shown in FIG. 3 and FIG. 4, the two electrodes 14 are both disposed on the surface of the side of the heating layer 12 away from the liquid conducting substance 11 and are respectively located on two sides of the oxide layer 13. The oxide layer 13 covers a part of the heating layer 12 that is not covered by the two electrodes 14, to ensure that heating layer 12 is completely covered by the oxide layer 13 and the two electrodes 14, and the heating layer 12 cannot contact the air to oxidize during atomization, so as to avoid the failure of the heating layer 12 due to the oxidation, thereby improving the stability of the atomization core 10 and prolonging the service life of the atomization core 10. The thickness of the two electrodes 14 is greater than the thickness of the oxide layer 13, which also facilitates the electrical connection between the power supply assembly 200 and the electrodes 14 through an electrical connector while ensuring good contact between the electrodes 14 and the heating layer 12, thereby improving the contact stability between the electrode 14 and the electrical connector. In addition, the thickness of the oxide layer 13 is relatively small, the oxide layer 13 absorbs less heat, the electric heating loss is low, and the atomization core 10 has a high temperature rise rate.

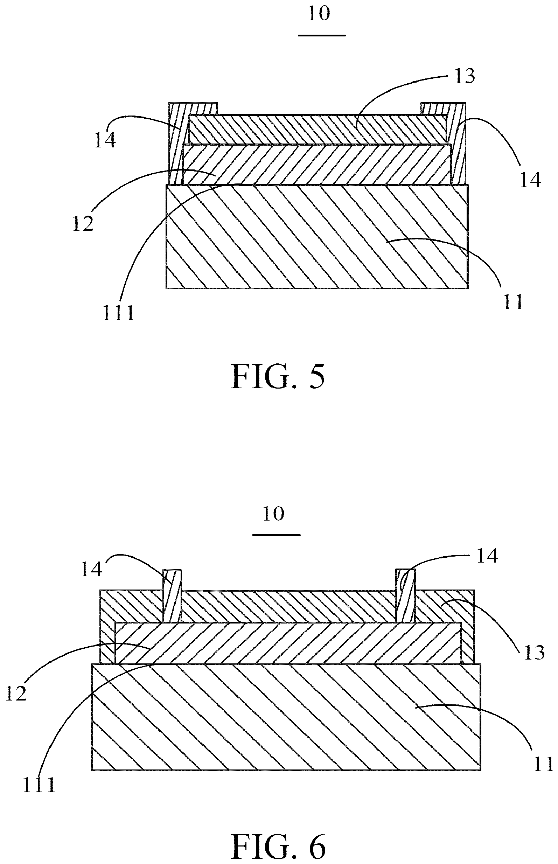

In a second embodiment, as shown in FIG. 5, the oxide layer 13 is disposed on the surface of the side of the heating layer 12 away from the liquid conducting substance 11, and the two electrodes 14 are disposed at intervals on the surface of the side of the oxide layer 13 away from the liquid conducting substance 11. The two electrodes 14 cover the part of the heating layer 12 that is not covered by the oxide layer 13, and the two electrodes 14 both contact the oxide layer 13, the heating layer 12, and the liquid conducting substance 11. The two electrodes 14 both contact the side of the heating layer (12) away from the liquid conducting substance 11,the side surfaces of the oxide layer 13, the side surfaces of the heating layer 12 and the side surfaces of the heating layer 12 to prevent a gap from existing between the electrode 14 and the oxide layer 13 when the electrode 14 is disposed on two sides of the oxide layer 13, which cannot completely isolate the contact between the air and the heating layer 12, resulting in the failure of the atomization core 10.

In a third embodiment, as shown in FIG. 6, the oxide layer 13 may also completely cover the surface of the heating layer 12 away from the liquid conducting substance 11 and the side surface of the heating layer 12. That is to say, the oxide layer 13 completely wraps the heating layer 12 to completely isolate the heating layer 12 from the air. Two through holes spaced apart from each other are disposed on the oxide layer 13 by forming a hole, and the two electrodes 14 are electrically connected to the heating layer 12 through the two through holes in the oxide layer 13, respectively. The two electrodes 14 are exposed from the surface of the side of the oxide layer 13 away from the liquid conducting substance 11 and are electrically connected to the power supply assembly 200.

Different from the prior art, the present disclosure discloses an atomization core, an atomizer, and an electronic atomization device. The atomization core in the present disclosure includes a liquid conducting substance, a heating layer, and an oxide layer. The liquid conducting substance has an atomization surface, the heating layer is disposed on the atomization surface of the liquid conducting substance, and the oxide layer is disposed on a surface of the heating layer away from the liquid conducting substance. The oxide layer is disposed on the surface of the heating layer away from the liquid conducting substance, so that the oxide layer protects the heating layer in the process of heating and atomization to avoid the failure of the heating layer due to oxidation in the process of the atomization, thereby the stability of the heating layer is improved, and the service life of the heating layer is further increased.

## Claims

1. A atomization core (10), comprising:
a liquid conducting substance (11), having an atomization surface (111);
a heating layer (12), disposed on the atomization surface (111) of the liquid conducting substance (11); and
an oxide layer (13), disposed on a surface of the heating layer (12) away from the liquid conducting substance (11).

2. The atomization core of claim 1, wherein the oxide layer (13) comprises at least one of aluminum oxide or silicon oxide.

3. The atomization core of claim 1, wherein the thickness of the oxide layer (13) ranges from 200 nm to 600 nm.

4. The atomization core of claim 1, wherein the oxide layer (13) is formed on the surface of the heating layer (12) away from the liquid conducting substance (11) through physical vapor deposition.

5. The atomization core of claim 1, further comprising two electrodes (14), wherein at least part of the two electrodes (14) are disposed on the surface of the heating layer (12) away from the liquid conducting substance (11); and the oxide layer (13) and the two electrodes (14) jointly cover the heating layer (12).

6. The atomization core of claim 5, wherein
the two electrodes (14) are disposed at intervals on the surface of the side of the heating layer (12) away from the liquid conducting substance (11) and are respectively located on two sides of the oxide layer (13), and the oxide layer (13) covers a part of the heating layer (12) that is not covered by the two electrodes (14).

7. The atomization core of claim 6, wherein
the two electrodes (14) both contact the oxide layer (13), the heating layer (12), and the liquid conducting substance (11).

8. The atomization core of claim 7, wherein
the two electrodes (14) both contact the surface of the side of the heating layer (12) away from the liquid conducting substance (11), the side surfaces of the oxide layer (13), the side surfaces of the heating layer (12) and the surface of the side of the liquid conducting substance (11) to prevent a gap from existing between the electrode (14) and the oxide layer (13) when the electrode (14) is disposed on two sides of the oxide layer (13).

9. The atomization core of claim 5, wherein
the oxide layer (13) completely cover the surface of the heating layer (12) away from the liquid conducting substance (11) and the side surface of the heating layer (12); wherein two through holes spaced apart from each other are disposed on the oxide layer (13) by forming a hole, and the two electrodes (14) are electrically connected to the heating layer (12) through the two through holes in the oxide layer (13), respectively.

10. The atomization core of claims 5 to 9, wherein the thickness of the oxide layer (13) is less than the thickness of the electrode (14).

11. The atomization core of claim 1, wherein the heating layer (12) is a porous heating film.

12. The atomization core of claim 1, wherein the oxide layer (13) is a porous structure.

13. The atomization core of claim 1, wherein the liquid conducting substance (11) is a porous ceramic substance or a dense substance having holes.

14. A atomizer (100), comprising a liquid storage cavity (90) configured to store an aerosol-forming substance and the atomization core (10) of any of claims 1 to 13, wherein the atomization core (10) is configured to absorb, heat, and atomize the aerosol-forming substance in the liquid storage cavity (90).

15. An electronic atomization device (300), comprising a power supply assembly (200) and the atomizer (100) of claim 14, wherein the power supply assembly (200) provides energy for the atomizer (100).
